# EUROPEAN PATENT APPLICATION

(11) **EP 4 450 064 A1**
(43) Date of publication of application: **23.10.2024**
(21) Application number: 24170265.3
(22) Date of filing: 15.04.2024
(51) Int. Cl.: A61K 9/00, A61K 31/00, A61K 47/10, A61K 47/12, A61K 47/14, A61K 47/26

(54) **APIXABAN COMPOSITIONS**

(30) Priority: 14.04.2023 IN 202341027698
(71) Applicant: Novick BioSciences Private Limited, Hyderabad 500037 (IN)
(72) Inventor: Jampana, Veera Venkata Satyanarayana Raju, 500032 Hyderabad (IN); Matam, Shiva Kumar, 500010 Secunderabad (IN); Sanagapati, Sirish Kumar, 500049 Hyderabad (IN); Jampana, Geetika, 500045 Hyderabad (IN)
(74) Representative: Nederlandsch Octrooibureau

(57) **Abstract**

The present invention relates to a stable aqueous solution composition of apixaban. The present disclosure also relates to a process for preparing stable aqueous solution compositions comprising apixaban and use of the composition.

## Description

### FIELD OF THE INVENTION

The present disclosure relates to a stable aqueous solution composition of apixaban. The present disclosure also relates to a process for preparing stable aqueous solution composition comprising apixaban and use of the composition thereof.

### BACKGROUND OF THE INVENTION

Apixaban is a factor Xₐ inhibitor and is chemically known as 4,5,6,7-tetrahydro-1-(4-methoxyphenyl)-7-oxo-6-[4-(2-oxo-1-piperidinyl)phenyl]-1H-pyrazolo[3,4-c]pyridine-3 carboxamide (CAS name) or 1-(4-methoxyphenyl)-7-oxo-6-[4-(2-oxo-1-piperidinyl)phenyl]-4,5,6,7-tetrahydro-1H-pyrazolo[3,4-c]pyridine-3-carboxamide (IUPAC name).

Apixaban in the form of a free base is approved and marketed as 2.5mg and 5mg tablets by Bristol Myers Squibb Co. (BMS) under the brand name ELIQUIS^{®} in the US.

Apixaban is indicated for the reduction of risk of stroke and systemic embolism in nonvalvular atrial fibrillation, prophylaxis of deep vein thrombosis following hip or knee replacement surgery, treatment of deep vein thrombosis, treatment of pulmonary embolism and reduction in the risk of recurrence of deep vein thrombosis and pulmonary embolism.

FDA approved label of ELIQUIS^{®} tablets allows administration by crushing the tablet and administering it by suspending it in water, 5% dextrose in water (D5W), apple juice or applesauce. However, the label mentions that such a suspension stable only upto 4 hours, thus indicating that apixaban is not stable in aqueous formulations.

Ellwanger et al (Analytical Quality by Design Approach for a Stability-Indicating Method to Determine Apixaban and its related impurities, Chromatographia, 83, pages 65-75 (2020*)*), Salakolousu et al (Identification, isolation, and structural characterization of novel,forced degradation products of apixaban using advanced analytical techniques, J Sep Sci, 2022 Nov;45(21): 3942-3954*)* and Secretan et al (A comprehensive study of apixaban's degradation pathways under stress conditions using liquid chromatography coupled to multistage mass spectrometry, RSC Adv., 2015, 5, 35586) are some of the references that discuss the instability of apixaban and the impurities that are generated in dosage forms. These references are incorporated herein by reference.

US9452134B2, assigned to BMS, discloses a liquid formulation comprising apixaban and a vehicle, wherein the vehicle comprises a solubilizer, preferably made up of a hydrophilic polymer and a combination of anionic and non-ionic surfactants. It also discloses that low aqueous solubility of apixaban (0.04 mg/mL) is a major hurdle in the development of a liquid formulation of apixaban. The patent claims a composition comprising a vehicle, wherein the water content of the vehicle is about 20% to about 30% w/w of the vehicle; nonionic surfactant content of the vehicle is about 11% to about 14% w/w of the vehicle; ionic surfactant content of the vehicle is about 0.2% to about 1% w/w of the vehicle; hydrophilic polymer content of the vehicle is about 1% to about 6% w/w of the vehicle; polyhydric alcohol content of the vehicle is about 31% to about 37% w/w of the vehicle; polyethylene glycol content of the vehicle is about 4% to about 6% w/w of the vehicle; and carbohydrate content of the vehicle is about 18% to about 22% w/w of the vehicle; wherein a solubility of apixaban in the vehicle is at least 0.50 mg/mL. While the BMS patent describes apixaban solutions that are bioavailable, it does not discuss stability, or issues therewith, for such aqueous solutions of apixaban. Further, the liquid formulation disclosed herein has a strength of only 0.4mg/ml, and a large volume of such a solution would have to be administered to deliver the approved dose of apixaban to a patient. Considering that the approved dose of apixaban is 5mg and 10mg for some of the conditions, 12.5ml and 25ml of such a solution would need to be administered. There is no teaching in this patent on preparing stable, concentrated solutions of apixaban that can contain approved doses of apixaban in lower volumes so that the dose can be administered easily. Given the known poor solubility of apixaban, it would not be routine for any skilled person to design a stable concentrated solution of apixaban for oral administration.

CN113384526A discloses an apixaban oral solution preparation comprising apixaban, a vector and other pharmaceutically acceptable auxiliary materials; the vector comprises water and a novel solubilizer, wherein the novel solubilizer is selected from one or more of diethylene glycol monoethyl ether, caprylic capric polyethylene glycol glyceride, and 15-hydroxystearic acid polyethylene glycol ester. It does not provide any teaching on stability of the composition.

Ionic surfactants suggested, for use in the prior art for enhancing solubility, such as sodium lauryl sulfate (SLS), have been reported to cause oral mucosal desquamation (See Esteves CV, de Campos WG, Paredes WEB, Nunes FD, Alves FA, Lemos CA. Could sodium lauryl sulfate be an irritant factor in oral mucosal desquamation? Int J Case Rep Images 2020; 11:101184Z01CE2020)*,* and are also known to irritate the eyes, cause dryness and itching of skin, and possibly gastric irritation.

Stable aqueous solution compositions of apixaban are advantageous over conventional solid dosage forms of apixaban, in terms of ease of administration to pediatric and geriatric patients, as well as in patients that are incapacitated and are confined to the bed. The stable solution compositions would also be amenable for administration by nasogastric (NG) tube, if required.

Therefore, there is a long felt need in the art for aqueous solutions of apixaban that are stable over a long period of time and which do not contain any irritants such as ionic surfactants.

### OBJECT OF THE INVENTION

It is an object of the invention to provide a stable aqueous solution composition of apixaban for overcoming the difficulties associated with administration of conventional solid dosage forms.

Another object of the invention is to provide a stable oral aqueous solution composition that is stable over a period of time.

Yet another object of the invention is to provide a stable aqueous solution composition free of ionic surfactants.

Further object of the invention is to use the stable aqueous solution composition in manufacture of a medicament in treating or preventing a disease or a condition such as thromboembolic disorder, stroke, and systemic embolism in nonvalvular atrial fibrillation, prophylaxis of deep vein thrombosis, deep vein thrombosis, and pulmonary embolism.

These and other objects and advantages of the invention will be apparent from the ensuing description.

### SUMMARY OF THE INVENTION

The present disclosure provides a stable aqueous apixaban solution formulation that contains an organic acid.

In an embodiment, the present disclosure provides a stable aqueous solution composition of apixaban that is free of ionic surfactants and contains an organic acid.

In one aspect of the present invention, there is provided an aqueous solution composition of apixaban, comprising
(a) 1 mg/ml of apixaban,
(b) a vehicle selected from one or more of water, polyoxyl 35 castor oil, macrogol-15-hydroxystearate, ethanol, propylene glycol, glycerine and sorbitol, preferably a vehicle comprising water in admixture with one or more of polyoxyl 35 castor oil, macrogol-15-hydroxystearate, ethanol, propylene glycol, glycerine and sorbitol,
(c) optionally, a preservative selected from methyl paraben, propyl paraben, methyl paraben sodium, propyl paraben sodium, methyl 4-hydroxy benzoate, propyl 4-hydroxy benzoate and mixtures thereof,
(d) an organic acid in an amount of about 0.01 mg/ml to about 10 mg/ml, wherein, preferably, upon storage at 40⁰C/75% RH for up to 6 months, the amount of apixaban in the composition is NLT 98%.

In another aspect of the present invention, there is provided an aqueous solution composition of apixaban solution formulation comprising
(a) 1 mg/ml of apixaban,
(b) a vehicle selected from one or more of water, polyoxyl 35 castor oil, macrogol-15-hydroxystearate, ethanol, propylene glycol, glycerine and sorbitol, preferably a vehicle comprising water in admixture with one or more of polyoxyl 35 castor oil, macrogol-15-hydroxystearate, ethanol, propylene glycol, glycerine and sorbitol
(c) optionally, a preservative selected from methyl paraben, propyl paraben, methyl paraben sodium, propyl paraben sodium, methyl 4-hydroxy benzoate, propyl 4-hydroxy benzoate and mixtures thereof,
(d) an organic acid in an amount of about 0.01 mg/ml to about 10 mg/ml, wherein upon storage at 40⁰C/75% RH for up to 6 months, impurity A in the composition is NMT 0.15%.

In one aspect of the present invention, there is provided a stable aqueous solution composition of apixaban comprising
(e) 1 mg/ml of apixaban,
(f) a vehicle selected from one or more of water, polyoxyl 35 castor oil, macrogol-15-hydroxystearate, ethanol, propylene glycol, glycerine and sorbitol,
(g) optionally, a preservative selected from methyl paraben, propyl paraben, methyl paraben sodium, propyl paraben sodium, methyl 4-hydroxy benzoate, propyl 4-hydroxy benzoate and mixtures thereof,
(h) an organic acid in an amount of about 0.01 mg/ml to about 10 mg/ml,
wherein upon storage at 40⁰C/75% RH for up to 6 months, the amount of apixaban in the composition is NLT 99.5%.

In another aspect of the present invention, there is provided a stable aqueous solution composition of apixaban comprising
(e) 1 mg/ml of apixaban,
(f) a vehicle selected from one or more of water, polyoxyl 35 castor oil, macrogol-15-hydroxystearate, ethanol, propylene glycol, glycerine and sorbitol,
(g) optionally, a preservative selected from methyl paraben, propyl paraben, methyl paraben sodium, propyl paraben sodium, methyl 4-hydroxy benzoate, propyl 4-hydroxy benzoate and mixtures thereof,
(h) an organic acid in an amount of about 0.01 mg/ml to about 10 mg/ml,
wherein upon storage at 40⁰C/75% RH for up to 6 months, impurity A in the composition is NMT 0.15%.

In yet another aspect, the present invention provides a stable aqueous solution composition of apixaban comprising: a) apixaban in an amount ranging from about 0.01% to about 20% w/w of the composition; b) water in an amount ranging from about 30% to about 99% w/w of the composition; and c) an alcohol in an amount ranging from about 0.001% to about 10% w/w of the composition, wherein the composition is substantially free of anionic surfactant and/or hydrophilic polymers.

The composition is stable for a time period in the range of from 15 days to 24 months, or more.

In another aspect of the present disclosure, the composition has a pH in the range of from about 3.5 to about 8.

In yet another aspect of the present disclosure, there is provided a process for preparing a stable aqueous solution composition of apixaban as disclosed herein, comprising the steps of: (i) adding apixaban to an aqueous solution of a non-ionic surfactant under stirring, at 60°C, to form a first solution; (ii) adding one or more polyhydric alcohols to this solution under stirring to obtain a second solution; (iii) adding an alcohol to the second solution under stirring to obtain a third solution; (iv) adding at least one of the excipient selected from a flavouring agent, an antioxidant, a sweetener, a preservative, a buffer, or combinations thereof to the third solution under stirring to obtain the oral liquid composition.

In further aspect of the present disclosure, there is provided a stable aqueous solution composition for use in manufacture of a medicament in treating or preventing a disease or a condition. The condition or the disease is selected from a group consisting of thromboembolic disorder, stroke, and systemic embolism in nonvalvular atrial fibrillation, prophylaxis of deep vein thrombosis, deep vein thrombosis, and pulmonary embolism. The composition is administered orally or through nasogastric tube or through gastronomy tube using a dosing syringe.

### DETAILED DESCRIPTION OF THE INVENTION

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

Unless the context indicates otherwise, it is specifically intended that the various features of the invention described herein can be used in any combination.

Moreover, the present disclosure also contemplates that in some embodiments of the invention, any feature or combination of features set forth herein can be excluded or omitted.

All publications, patent applications, patents, and other references mentioned herein are incorporated by reference in their entirety for all purposes.

As used herein, "a," "an," or "the" can mean one or more than one.

Furthermore, the term "about," as used herein, when referring to a measurable value such as an amount of a compound or agent of this invention, dose, time, temperature, and the like, is meant to encompass variations of ±10% of the specified amount.

The term composition(s), product(s), preparation(s) and formulation(s) have been used interchangeably.

The term "aqueous solution composition of apixaban" is used herein to indicate that the composition has the form of an aqueous system in which apixaban is dissolved, as will be readily understood by those skilled in the art based on the present disclosure.

The term "comprising", which is synonymous with "including", "containing", or "characterized by" herein defined as being inclusive or open-ended and does not exclude additional, unrecited elements or method steps, unless the context clearly requires otherwise.

Also as used herein, "and/or" refers to and encompasses any and all possible combinations of one or more of the associated listed items, as well as the lack of combinations when interpreted in the alternative ("or").

The term "excipient" as used herein refers to a component that can be added in the composition, which is physiologically tolerable and does not typically produce allergic or similar untoward reaction. The excipient also provides improved stability and therapeutic enhancement to the composition. The excipients may be added to the composition to facilitate manufacturing process, enhance stability, control release, enhance product characteristics, enhance bioavailability, enhance patient acceptability, or the like. The term "excipient" and "pharmaceutically acceptable excipient" shall be used interchangeably.

The United States Pharmacopoeia (USP) defines stability as "the ability of a product to retain its characteristics that it possessed during its manufacturing (physical, chemical, microbiological, therapeutic properties) within specified limits throughout its period of storage and use". According to the ICH guidelines, pharmaceutical stability testing is defined as "systematic experiments conducted on pharmaceutical products to understand and provide evidence how the quality of a drug product varies under the influence of a variety of environmental factors such as temperature, humidity and light, and to set a re-test period for the drug, or a shelf life for the drug product and recommend good storage conditions". In view of these, degradation products in a drug product are required to be evaluated and reported to the regulatory agencies. Further, USFDA requires formulations to be stable for a minimum period of 12 months (i.e. 48 weeks) at recommended storage condition, for approving the same for commercial use. For products to be commercially marketed in the US, there is an expectation that the products will comply with the standard throughout the shelf life.

Thus, the composition must have an acceptable level of impurity. The term "acceptable level of impurity" refers to the impurities in drug products not exceeding the permitted daily exposure (PDE), and/or impurities within limits defined by ICH for pharmaceutical products. "Stable", as used herein, refers to physical and chemical stability of apixaban in the aqueous solution, wherein the assay values remain within ±10% of the initial assay value, and the total impurities in the composition remain at less than 0.5% w/w, upon storage of the composition at 40⁰C/75% relative humidity (RH) for up to 6 months, and/or at 25°C/60% RH for up to 6 months.

Whenever, in this document, the amount of apixaban and/or amounts of impurities in the composition after storage (under certain conditions) are given, typically as a percentage amount (% w/w), this denotes the percentage relative to the total amount of apixaban incorporated in the composition, as will be readily understood by those skilled in the art, based on the present disclosure. Hence, the indication that the amount of apixaban in the composition is NLT 98%, means that, after storage under the conditions indicated, the amount of apixaban is not less than 98% (on a w/w basis) of the amount of apixaban originally incorporated in the composition. Similarly, the indication that impurity A in the composition is NMT 0.15%, means that, after storage under the conditions indicated, the amount of impurity A is not more than 0.15% (on a w/w basis) of the amount of apixaban originally incorporated.

The term 'stable' also encompasses a composition that has a shelf-life of at least 12 months. The term "shelf life" refers to the amount of time the pharmaceutical composition may be stored without loss of potency and/or performance profile. In some embodiments of the present disclosure, shelf life refers to the amount of time the composition may be stored with loss of not more than about 5%, preferably not more than about 4%, preferably note more than about 3%, preferably not more than about 2% or still preferably not more than about 1% of the potency and/or performance, when stored at room temperature of 25⁰C and 60% relative humidity. The stable composition provided herein is designed to have a shelf life of at least about 12 months, preferably about 15 months, more preferably about 18 months, even more preferably about 24 months.

The term "vehicle" as used herein refers to a solvent or liquid present in the composition. The composition of the present invention comprises vehicle in a range of about 10 to about 80% w/w with respect to the total weight of the composition. The vehicle comprises one or more of water, polyoxyl 35 castor oil, macrogol-15-hydroxystearate, ethanol, propylene glycol, glycerine and sorbitol.

Ratios, concentrations, amounts, and other numerical data may be presented herein in a range format. It is to be understood that such range format is used merely for convenience and brevity and should be interpreted flexibly to include not only the numerical values explicitly recited as the limits of the range, but also to include all the individual numerical values or sub-ranges encompassed within that range as if each numerical value and sub-range is explicitly recited. For example, a range of 0.001 to 90% w/w should be interpreted to include not only the explicitly recited limits of 0.001% to 90% but also to include sub- ranges, such as 0.05% to 40%, 0.1% to 87% and so forth, as well as individual amounts, including fractional amounts, within the specified ranges, such as 0.022%, 0.01%, 0.58%, 0.9%, 1.39%, 5.4%, 22.6% for example.

As discussed in the background, solubility of apixaban is a key hurdle to developing a stable oral dosage form of apixaban, especially for pediatric and geriatric populations, as well as for patients that are hospitalized and/or are bedridden. Also, existing liquid formulations are not stable over a longer period of time. As is known from Eliquis^{®} label, a suspension of crushed tablet is usable for only upto 4 hours, and as is shown below, such a suspension loses its potency (as indicated by fall in assay) when administered by NG tube.

In one embodiment, "apixaban" according to the present disclosure includes, but is not limited to, apixaban free base and its acceptable salts, ethers, esters, polymorphs, prodrugs, isomers, derivatives thereof.

In another embodiment, apixaban used is in the form of a free base.

In yet another embodiment of the present disclosure, there is provided the stable aqueous liquid composition comprising apixaban in the range of about 0.1mg to about 50 mg, preferably about 1 mg to about 10 mg, and more preferably about 1 mg to about 5 mg.

In another embodiment of the present invention, there is provided the stable aqueous composition comprising:
a) apixaban or a pharmaceutically acceptable salt thereof,
b) a non-ionic surfactant;
c) an alcohol;
d) optionally an excipient, and
e) water.

In another embodiment of the present invention, there is provided the stable aqueous composition comprising:
a) apixaban or a pharmaceutically acceptable salt thereof,
b) water; and
c) an excipient.

In another embodiment of the present invention, there is provided the stable aqueous composition comprising:
a) apixaban or a pharmaceutically acceptable salt thereof;
b) an alcohol;
c) water; and
d) optionally an excipient.

In another embodiment of the present invention, there is provided the stable aqueous composition comprising:
a) apixaban or a pharmaceutically acceptable salt thereof;
b) a vehicle
c) water; and
d) optionally an excipient.

In an embodiment of the present invention, there is provided a stable aqueous composition comprising:
a) apixaban in a range of about 0.01% to about 1% w/w with respect to the composition;
b) water in a range of about 30% to about 99% w/w with respect to the composition;
c) an alcohol in a range of about 0.001% to about 10% w/w with respect to the composition;
d) a non-ionic surfactant in a range of about 0.1% to about 30% w/w with respect to the composition, and
e) an organic acid in a range of about 0.001% to about 10% w/w with respect to the composition.

In an embodiment of the present invention, there is provided a stable aqueous composition comprising :
a) apixaban in a range of about 0.01% to about 1% w/w with respect to the composition;
b) water in a range of about 40% to about 99% w/w with respect to the composition;
c) alcohol in a range of about 0.001% to about 10% w/w with respect to the composition;
d) a non-ionic surfactant in a range of about 0.1% to about 30% w/w with respect to the composition;
e) a polyhydric alcohol in a range of about 0.001% to about 60% w/w with respect to the composition;
f) a flavoring agent in a range of about 0.1% to about 10% w/w with respect to the composition;
g) an antioxidant in a range of about 0.1% to about 10% w/w with respect to the composition;
h) a sweetener in a range of about 0.1% to about 50% w/w with respect to the composition;
i) a preservative in a range of about 0.1% to about 10% w/w with respect to the composition; and
j) an organic acid in a range of about 0.001% to about 10% w/w with respect to the composition.

In a further embodiment of the present disclosure, apixaban may be present in crystalline form or amorphous form.

In another embodiment, the stable aqueous solution according to the present disclosure comprises apixaban in a range of about 0.01% to about 1% w/w, preferably in a range of about 0.025 % to about 1 %, and still more preferably in a range of about 0.05 % to about 0.5 %, e.g. about 0.1 %.

A non-ionic surfactant as referred to herein is a non-ionizable surface-active agent. Structurally, nonionic surfactants combine uncharged hydrophilic and hydrophobic group that make them effective in wetting and spreading and as emulsifiers and foaming agents. Non-ionic surfactants that can be used in the stable aqueous solution compositions of the present disclosure are selected from a group comprising poly (alkylene-oxide) block copolymers, oligomeric alkyl-ethylene oxides, alkyl-phenol poly-ethylenes, sorbitan esters and mixtures thereof. Nonlimiting examples include polyoxyethylene sorbitan fatty acid esters (polysorbates such as polysorbate 20, polysorbate 40, polysorbate 60, and polysorbate 80); polyoxyethylene- polyoxypropylene block co-polymer (poloxamers such as poloxamer 124,poloxamer 188, poloxamer 237, poloxamer 338, and poloxamer 407); polyoxyethylene esters of stearic acid (such as polyoxyl 15 hydroxystearate); polyoxyethylene castor oil derivatives (such as polyoxyl 35 castor oil and polyoxyl 40 hydrogenated castor oil); polyoxyglycerides are polyethylene glycol-8 caprylic/capric glycerides; fatty alcohols such as lauryl, cetyl and stearyl alcohols; glyceryl esters such as the naturally occurring mono-, di-, and tri-glycerides; fatty acid esters of fatty alcohols; polyglycolized glycerides such as Gelucire; vitamin E polyethylene glycol succinate, and macrogol 15 hydroxystearate, and mixtures thereof. In a preferred embodiment of the present disclosure, the surfactant is a nonionic surfactant. In a preferred embodiment of the present disclosure, the surfactant is selected from the group consisting of polyoxyethylene castor oil derivatives (such as polyoxyl 35 castor oil and polyoxyl 40 hydrogenated castor oil) and polyoxyethylene esters of stearic acid (such as polyoxyl 15 hydroxy stearate).

In another embodiment, the stable aqueous solution according to the present disclosure comprises the non-ionic surfactant in a range of 0.1% to about 30% w/w with respect to the composition, such as in a range of about 0.5 % to about 20% w/w, preferably in a range of about 1 % to about 15% w/w of the composition, more preferably in a range of about 2.5 % to about 12.5 %, and still more preferably in a range of about 4 % to about 10 %, e.g. about 8 %.

In an embodiment of the present disclosure, there is provided the stable aqueous solution composition as disclosed herein, wherein the alcohol is selected from a group comprising ethanol, 2-(2-ethoxyethoxy) ethanol, benzyl alcohol, glycerol, polyethylene glycol 200, polyethylene glycol 300, polyethylene glycol 400, or combinations thereof. In a preferred embodiment of the present disclosure, the alcohol is ethanol.

In another embodiment, the stable aqueous solution according to the present disclosure comprises the alcohol in a range of about 0.1% to about 30% w/w with respect to the composition, such as in a range of about 1 % to about 25% w/w, preferably in a range of about 2.5 % to about 20% w/w of the composition, more preferably in a range of about 5 % to about 15 %, and still more preferably in a range of about 7.5 % to about 12.5 %, e.g. about 10%.

In one embodiment of the present disclosure, there is provided the stable aqueous solution composition as disclosed herein, wherein the polyhydric alcohol is selected from a group comprising glycerin, propylene glycol, sorbitol, mannitol, maltitol, and mixtures thereof. In a preferred embodiment of the present disclosure, the polyhydric alcohol is glycerin, propylene glycol, sorbitol or a mixture thereof. In a particularly preferred embodiment of the present disclosure, the polyhydric alcohol is a mixture of glycerin, propylene glycol and sorbitol, wherein, e.g. the ratio of propylene glycol to glycerin is within the range of 25:6 to about 40:6, such as about 30:6 to about 34:6 and/or wherein the ratio of propylene glycol to sorbitol is about 25:12 to about 40:12, such as about 30:12 to about 34:12.

In another embodiment, the stable aqueous solution according to the present disclosure comprises the polyhydric alcohol in a range of about 5% to about 70% w/w with respect to the composition, such as in a range of about 25 % to about 65% w/w, preferably in a range of about 40 % to about 60 % w/w of the composition, more preferably in a range of about 45 % to about 55 %, e.g. about 48 %.

In an embodiment of the present disclosure, there is provided the stable aqueous solution composition as disclosed herein, wherein an organic acid is selected from the group comprising citric acid, malic acid, tartaric acid, phosphoric acid, glutamic acid, lactic acid, succinic acid, pimelic acid, adipic acid, ascorbic acid, fumaric acid, acetic acid or combinations thereof. In a preferred embodiment of the present disclosure, the organic acid is selected from the group consisting of malic acid, citric acid and tartaric acid.

In another embodiment, the stable aqueous solution according to the present disclosure comprises the organic acid in a range of about 0.001% to about 5% w/w with respect to the composition, such as in a range of about 0.01 % to about 2.5% w/w, preferably in a range of about 0.02 % to about 1.5 % w/w of the composition, more preferably in a range of about 0.04 % to about 1 %, and still more preferably in a range of about 0.05 % to about 0.5 %. In another embodiment, the organic acid is citric acid, which is present in a range of about 0.001% to about 2.5% w/w with respect to the composition, such as in a range of about 0.01 % to about 1% w/w, preferably in a range of about 0.02 % to about 0.5 % w/w of the composition, more preferably in a range of about 0.04 % to about 0.1 %, and still more preferably in a range of about 0.05 % to about 0.08 %, e.g. in an amount of about 0.065%. In another embodiment, the organic acid is malic acid or tartaric acid, which is present in a range of about 0.001% to about 5% w/w with respect to the composition, such as in a range of about 0.01 % to about 2.5% w/w, preferably in a range of about 0.05 % to about 2 % w/w of the composition, more preferably in a range of about 0.1 % to about 1 %, and still more preferably in a range of about 0.25 % to about 0.8 %, e.g. in an amount of about 0.4 % or about 0.675%.

In an embodiment of the present disclosure, there is provided the stable aqueous solution composition as disclosed herein, further comprising 0.1% to about 10% (w/w) of a preservative. The preservative is selected from a group comprising methyl paraben, ethyl paraben, propyl paraben, benzoic acid, sorbic acid, sodium benzoate, methyl 4-hydroxy benzoate and propyl 4-hydroxy benzoate. The preservative may include one or more of the listed preservatives to effectively preserve the apixaban oral solution.

In an embodiment of the present disclosure, there is provided the stable aqueous solution composition as disclosed herein, wherein the flavoring agent is selected from orange flavor, cherry flavor, strawberry flavor, bubblegum flavor, grape flavor, lemon flavor, banana flavor, mango flavor, pineapple flavor, mint flavor, mixed fruit flavor, vanilla flavor, tutti-fruity flavor, fruit punch flavor, and mixtures thereof.

In another embodiment of the present disclosure, there is provided the stable aqueous solution composition as disclosed herein, wherein the antioxidant is selected from the group comprising ascorbic acid, butylated hydroxy anisole, butylated hydroxytoluene, vitamin E, vitamin E PEG 1000 succinate, and mixtures thereof.

In yet another embodiment of the present disclosure, there is provided the stable aqueous solution composition as disclosed herein, wherein the sweetener is selected from artificial sweeteners, such as sucralose, aspartame, acesulfame potassium, saccharin sodium, high fructose corn syrup; carbohydrates, such as sucrose, fructose, lactose, dextrose; and mixtures thereof.

In further embodiment of the present disclosure, there is provided the stable aqueous solution composition as disclosed herein, wherein the buffer may be selected from a group comprising malic acid, tartaric acid, phosphoric acid, glutamic acid, lactic acid, succinic acid, pimelic acid, adipic acid, ascorbic acid, tosylic acid, benzenesulfonic acid, suberic acid, alginic acid, fumaric acid, acetic acid, citrate buffer, acetate buffer, phosphate buffer, and mixtures thereof.

In an embodiment of the present disclosure, there is provided the stable aqueous solution composition as disclosed herein, wherein apixaban is in a range of about 0.01% to about 20% w/w with respect to the composition; water is in a range of about 10% to about 99% w/w with respect to the composition; the nonionic surfactant is in a range of about 0.1% to about 30% w/w with respect to the composition; the alcohol is in a range of about 0.001% to about 10% w/w with respect to the composition; the flavoring agent is in a range of about 0.1% to about 10% w/w with respect to the composition; an organic acid is in a range of about 0.001% to about 10% w/w with respect to the composition; the polyhydric alcohol is in a range of about 0.001% to about 60% w/w with respect to the composition; the antioxidant is in a range of about 0.1% to about 10% w/w with respect to the composition; the sweetener is in a range of about 0.1% to about 50%w/w with respect to the composition, and the preservative is in a range of about 0.1% to about 10% w/w with respect to the composition.

In an embodiment of the present disclosure, there is provided the stable aqueous solution composition as disclosed herein, wherein the total level of ionic surfactants of the composition is less than 0.1 % w/w with respect to the composition, preferably at a total amount less than 0.05 %, less than 0.01 %, less than 0.005 %, less than 0.001 %, less than 0.0005 % or less than 0.0001 %. In an embodiment of the present disclosure, there is provided the stable aqueous solution composition as disclosed herein, wherein the solution is substantially free of ionic surfactants. In an embodiment of the present invention, there is provided the stable aqueous solution as disclosed herein wherein the composition is free of ionic surfactant. In an embodiment of the present invention, there is provided the stable aqueous solution as disclosed herein wherein the level of ionic surfactant in the composition is 0 %.

In yet another aspect of the present disclosure, there is provided a process for preparing a stable aqueous solution composition of apixaban as disclosed herein, comprising the steps of: (i) adding apixaban to an aqueous solution of a non-ionic surfactant under stirring at 60°C, to form a first solution; (ii) adding one or more polyhydric alcohols to the first solution under stirring to obtain a second solution; (iii) adding an alcohol to the second solution under stirring to obtain a third solution; (iv) adding at least one of the excipient selected from a flavouring agent, an antioxidant, a sweetener, a preservative, a buffer, or combinations thereof to the third solution under stirring to obtain the oral liquid composition.

In embodiments of the present disclosure, modulation of the pH is done to provide a better impurity profile, i.e. compositions with lower impurities. Preferably, the pH is adjusted in the range of about 3.50 to about 8.0. In the exemplary stability studies, the main apixaban degradants are Impurity A, Impurity B, Impurity C, Impurity D and Impurity E. Structural details of the impurities, as well their specifications are provided in table A below.

**Table A:**

| **Type of impurity** | **IUPAC Chemical Name** | **Chemical Structure** | **Specification** |
|---|---|---|---|
| Impurity A | 1-(4-methoxyphenyl)-7-oxo-6-[4-(2-oxo-1-piperdyl)phenyl]-4,5 dihydropyrazolo[3,4-c]pyridine-3-carboxylic acid (ABN-Acid) | 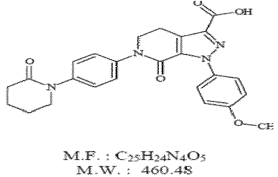 | NMT 0.5% |
| Impurity B | N-formyl-1-(4-methoxy phenyl)-7-oxo-6-[4-(2- oxo-1-piperidyl) phenyl]-4,5-dihydro pyrazolo[3,4-c] pyridine-3-carboxamide (N-Formyl impurity) | 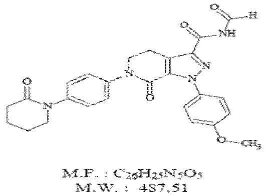 | NMT 0.5% |
| Impurity C | Methyl 1-( 4-methoxy pheny1)-7-oxo-6-[4-(2-oxo-1-piperidyl) phenyl]-4,5-dihydro pyrazolo[3,4-c] pyridine-3-carboxylate (Methyl ester impurity) | 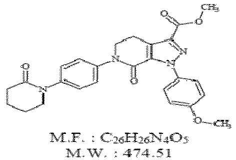 | NMT 0.5% |
| Impurity D | Ethyl-1-(4-methoxy phenyl)-7-oxo-6-[4-(2-oxo-1-piperidyl) phenyl]-4,5-dihydro pyrazolo [3,4-c] pyridine-3-carboxylate (ABN-1) | 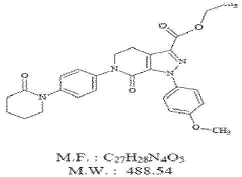 | NMT 0.5% |
| | | | |
| Impurity E | 6-[4-[(5-amino-5-oxopentyl) amino] phenyl]- 1-(4-methoxyphenyl)-7-oxo-4,5-dihydro pyrazolo [3,4-c] pyridine-3-carboxamide | 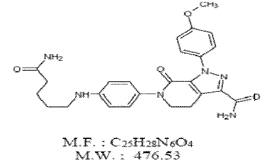 | NMT 0.5% |

| | | | |
|---|---|---|---|
| NMT : Not More Than | | | |

In an embodiment of the present disclosure, there is provided the stable aqueous solution composition comprising 0.01 to 20% (w/w) of apixaban or a pharmaceutically acceptable salt thereof as the sole active ingredient, 10% to 80% (w/w) of a vehicle selected from a group comprising water, non-ionic surfactants, alcohols, polyhydric alcohols and mixtures thereof; and 0.001% to about 10% w/w of an organic acid, wherein upon storage at 40⁰C/75% RH for up to 6 months, the amount of apixaban in the composition is not less than about 99.5%w/w, and the amount of impurity A in the composition is not more than about 0.15%w/w.

In another embodiment of the present disclosure, there is provided the stable aqueous solution composition comprising 0.01 to 20% (w/w) of apixaban or a pharmaceutically acceptable salt thereof as the sole active ingredient, 10% to 80% (w/w) of a vehicle selected from a group comprising water, non-ionic surfactants, alcohols, polyhydric alcohols and mixtures thereof; and 0.001% to about 10% w/w of an organic acid, wherein upon storage at 40⁰C/75% RH for up to 6 months, the amount of apixaban in the composition is not less than about 99.5%w/w, and the amount of impurity B in the composition is not more than about 0.15%w/w.

In yet another embodiment of the present disclosure, there is provided the stable aqueous solution composition comprising 0.01 to 20% (w/w) of apixaban or a pharmaceutically acceptable salt thereof as the sole active ingredient, 10% to 80% (w/w) of a vehicle selected from a group comprising water, non-ionic surfactants, alcohols, polyhydric alcohols and mixtures thereof; and 0.001% to about 10% w/w of an organic acid, wherein upon storage at 40⁰C/75% RH for up to 6 months, the amount of apixaban in the composition is not less than about 99.5%w/w, and the amount of impurity C in the composition is not more than about 0.15%w/w.

In a further embodiment of the present disclosure, there is provided the stable aqueous solution composition comprising 0.01 to 20% (w/w) of apixaban or a pharmaceutically acceptable salt thereof as the sole active ingredient, 10% to 80% (w/w) of a vehicle selected from a group comprising water, non-ionic surfactants, alcohols, polyhydric alcohols and mixtures thereof; and 0.001% to about 10% w/w of an organic acid, wherein upon storage at 40⁰C/75% RH for up to 6 months, the amount of apixaban in the composition is not less than about 99.5%w/w, and the amount of impurity D in the composition is not more than about 0.15%w/w.

Furthermore, in another embodiment, the present disclosure also provides a stable aqueous solution composition comprising 0.01 to 20% (w/w) of apixaban or a pharmaceutically acceptable salt thereof as the sole active ingredient, 10% to 80% (w/w) of a vehicle selected from a group comprising water, non-ionic surfactants, alcohols, polyhydric alcohols and mixtures thereof; and 0.001% to about 10% w/w of an organic acid, wherein upon storage at 40⁰C/75% RH for up to 6 months, the amount of apixaban in the composition is not less than about 99.5%w/w, and the amount of any known impurity in the composition is not more than about 0.15% w/w.

In another embodiment of the present disclosure, the stable aqueous composition is suitable for oral administration and/or administration through a nasogastric tube and/or gastronomy tube using a dosing syringe.

In another embodiment of the present disclosure, the stable aqueous composition comprising apixaban is used in the treatment of a thromboembolic disorders in a patient in need thereof.

In another embodiment of the present disclosure, the stable aqueous composition comprising apixaban is used for the reduction of risk of stroke and systemic embolism in nonvalvular atrial fibrillation, prophylaxis of deep vein thrombosis following hip or knee replacement surgery, treatment of deep vein thrombosis, treatment of pulmonary embolism and reduction in the risk of recurrence of deep vein thrombosis and pulmonary embolism.

In an embodiment of the present disclosure, there is provided a stable aqueous composition as disclosed herein for use in manufacture of a medicament in treating or preventing a disease or a condition.

In an embodiment of the present disclosure, there is provided a composition as disclosed herein for use in manufacture of a medicament in treating or preventing a disease or a condition selected from the group consisting of thromboembolic disorder, stroke, and systemic embolism in nonvalvular atrial fibrillation, prophylaxis of deep vein thrombosis, deep vein thrombosis, and pulmonary embolism.

In an embodiment of the present disclosure, there is provided a method oftreating or preventing a disease/ or a condition, the method comprising: administering the composition as disclosed herein to a subject in need thereof.

In an embodiment of the present disclosure, there is provided a method of treating or preventing a disease/ or a condition, the method comprising: administering the composition as disclosed herein to a subject in need thereof, wherein the disease or the condition is selected from the group consisting of thromboembolic disorder, stroke, and systemic embolism in nonvalvular atrial fibrillation, prophylaxis of deep vein thrombosis, deep vein thrombosis, and pulmonary embolism.

In an embodiment of the present disclosure, there is provided a method of treating or preventing a disease/ or a condition as disclosed herein, wherein the composition as disclosed herein is administered orally or through nasogastric tube or through gastronomy tube using a dosing syringe.

Although the subject matter has been described in considerable detail with reference to certain examples and implementations thereof, other implementations are possible.

The present invention is further illustrated by reference to the following examples which are for illustrative purpose only and do not limit the scope of the invention in any manner.

### EXAMPLES

### Example 1

The compatibility of apixaban was confirmed with organic acids such as citric acid, malic acid and tartaric acid at a temperature of about 60^{O}C over a time period of 30 days. Specifically, the impurities were analyzed by HPLC on the initial day, 15^{th} day and the 30^{th} day. The results of the same are included in Table 1 below.

**Table 1: Apixaban and organic acid compatibility study**

| **With Citric acid** | | | | **With Malic acid** | | | **With Tartaric acid** | | |
|---|---|---|---|---|---|---|---|---|---|
| **Name of the Impurity** | **Initial** | **60°C** | | **Initial** | **60°C** | | **Initial** | **60°C** | |
| | | **15 days** | **30 days** | | **15 days** | **30 days** | | **15 days** | **30 days** |
| **Impurity-A** | 0.00 | 0.50 | 0.74 | 0.07 | 0.03 | ND | 0.08 | 0.04 | 0.07 |
| **Impurity-B** | 0.00 | 0.00 | ND | ND | ND | ND | ND | ND | ND |
| **Impurity-C** | 0.05 | 0.00 | 0.05 | 0.05 | 0.03 | 0.02 | 0.02 | 0.02 | 0.02 |
| **Impurity-D** | 0.00 | 0.00 | 0.17 | ND | ND | ND | ND | ND | ND |
| **Unknown impurity @0.90 RRT** | 0.49 | 2.42 | 1.25 | ND | 0.06 | 0.06 | ND | 0.05 | 0.02 |
| **Total Impurities** | 0.69 | 3.41 | 2.86 | 0.21 | 0.21 | 0.35 | 0.14 | 0.19 | 0.19 |

| | | | | | | | | | |
|---|---|---|---|---|---|---|---|---|---|
| ND: Not detected | | | | | | | | | |

Based on the data shown in Table 1, malic acid and tartaric acid seem to provide a better control of unknown impurity at RRT 0.90 and total impurities.

### Example 2

Table 2 provides an oral liquid composition comprising apixaban.

**Table 2**

| **Ingredient** | **Quantity/mL** |
|---|---|
| Apixaban | 1 mg |
| Polyoxyl 35 Castor oil | 80 mg |
| Ethanol | 100 µL |
| 1,2-Propylene glycol | 300 µL |
| Glycerin | 60 µL |
| Sorbitol Solution (Neosorb 70/02B) | 120 µL |
| Sodium Benzoate | 1 mg |
| Citric acid | 4.5 mg |
| Banana flavor | 1 mg |
| Purified water | Q.s to 1 mL |

Polyoxyl 35 castor oil (non-ionic surfactant vehicle) was added, in the quantity mentioned in table 2 to purified water and heated to 60°C to obtain an aqueous solution. To this, Apixaban was added and stirred to form a clear solution. Thereafter, ethanol (alcohol-vehicle) was added to the clear solution and allowed to cool to 45°C. To this solution, sodium benzoate (preservative) and citric acid (organic acid) were added sequentially and stirred to form a clear solution. Banana flavor (flavoring agent) was then added to the solution and stirred to form a clear solution. The final volume was made up using purified water. The pH of final solution was checked and found to be 3.50.

### Example 3

Table 3 provides an oral liquid composition comprising apixaban. The composition was obtained by a process similar to that disclosed in Example 2 above. The pH of final solution was checked and found to be 3.50.

**Table 3**

| **Ingredient** | **Quantity/mL** |
|---|---|
| Apixaban | 1 mg |
| Polyoxyl Hydroxy stearate | 40 mg |
| Ethanol | 100 µL |
| 1,2-Propylene glycol | 340 µL |
| Glycerin | 60 µL |
| Sorbitol Solution (Neosorb 70/02B) | 120 µL |
| Sodium Benzoate | 1 mg |
| Citric acid | 4.5 mg |
| Banana flavor | 1 mg |
| Bitter masker | 1 mg |
| Purified water | Q.s to 1 mL |

### Example 4

The effect of pH on the formation of degradants in apixaban compositions of Example 2 and Example 3 was tested by storing the samples for up to 3 months.

Table 4.1 below provides the assay, pH and impurity profile of apixaban oral liquid compositions prepared according to Example 2 after storing at 40°C/75% RH for upto 3 months, at 30°C/75% RH for 3 months and at 25°C/60%RH for 3 months.

**Table 4.1: Stability data of composition of Example 2**

| | **Example 2 (with Polyoxyl 35 Castor oil)** | | | | | |
|---|---|---|---|---|---|---|
| | **40°C/75%RH** | | | | **30°C/75%RH** | **25°C/60%RH** |
| | **Initial** | **1M** | **2M** | **3M** | **3M** | **3M** |
| **Assay** | 97.4 | 96.4 | 96.5 | 94.4 | 96.6 | 97.2 |
| **pH** | 3.59 | 3.62 | 3.66 | 3.61 | 3.60 | 3.62 |
| **Related Substances** | | | | | | |
| **Impurity-A** | ND | 0.10 | ND | 0.05 | 0.09 | 0.09 |
| **Impurity-B** | ND | ND | ND | ND | ND | ND |
| **Impurity-C** | 0.02 | 0.03 | 0.02 | 0.03 | 0.03 | 0.03 |
| **Impurity-D** | ND | ND | ND | ND | ND | ND |
| **Unknown impurity @RRT 0.91** | 0.13 | 0.18 | 0.22 | 0.20 | 0.16 | 0.12 |
| **Total Impurities** | 0.19 | 0.33 | 0.32 | 0.31 | 0.31 | 0.26 |

The composition was found to have not more than 0.5% of total impurities when stored for 3 months at 40°C/75% RH, at 30°C/75% RH and at 25°C/60%RH. Further, other impurities are also within the acceptable range as provided in Table A above.

Table 4.2 given below provides the assay, pH and impurity profile of apixaban oral liquid composition prepared according to Example 3, after storing at 40°C/75% RH for upto 6 months, at 30°C/75% RH for 3 months and at 25°C/60%RH for 3 months and 6 months respectively.

**Table 4.2: Stability data of composition of Example 3**

| | **Example 3 (With Polyoxyl-15-hydroxy stearate)** | | | | | | | |
|---|---|---|---|---|---|---|---|---|
| | **40°C/75%RH** | | | | | **30°C/75%RH** | **25°C/60%RH** | |
| | **Initial** | **1M** | **2M** | **3M** | **6M** | **3M** | **3M** | **6M** |
| **Assay** | 96.7 | 96.4 | 97.6 | NA | NA | NA | NA | NA |
| **pH** | 3.78 | 3.82 | 3.86 | 3.80 | 3.75 | 3.81 | 3.84 | 3.76 |

| **Related Substances** | | | | | | | | |
|---|---|---|---|---|---|---|---|---|
| **Impurity-A** | ND | 0.08 | ND | 0.04 | 0.07 | 0.01 | 0.01 | 0.01 |
| **Impurity-B** | ND | ND | ND | ND | ND | ND | ND | ND |
| **Impurity-C** | 0.03 | 0.03 | 0.03 | 0.03 | 0.02 | 0.03 | 0.03 | 0.03 |
| **Impurity-D** | ND | ND | ND | ND | ND | ND | ND | ND |
| **Unknown impurity @ RRT 0.71** | ND | ND | 0.17 | 0.33 | 0.27 | 0.13 | 0.08 | 0.11 |
| **Unknown impurity @ RRT 0.91** | 0.06 | 0.09 | 0.15 | 0.20 | 0.26 | 0.11 | 0.11 | 0.11 |
| **Total Impurities** | 0.09 | 0.27 | 0.38 | 0.76 | 0.74 | 0.40 | 0.34 | 0.26 |

| | | | | | | | | |
|---|---|---|---|---|---|---|---|---|
| NA : Not available ND :Not detected | | | | | | | | |

### Example 5

Apixaban aqueous solutions with polyoxyl 35 castor oil and sodium salts of methyl paraben and propyl paraben is provided in Table 5 below, with a target pH of 5.50 - 7.50.

**Table 5**

| **Ingredient** | **Quantity/mL** | |
|---|---|---|
| | **Composition 5A** | **Composition 5B** |
| Apixaban | 1 mg | 1 mg |
| Polyoxyl 35 castor oil | 80 mg | 80 mg |
| Ethanol | 100 µL | 100 µL |
| Propylene Glycol Vegetable | 300 µL | 300 µL |
| Glycerin | 60 µL | 60 µL |
| Sorbitol solution (Neosorb 70/02B) | 120 µl | 120 µl |
| Methyl 4-hydroxy benzoate sodium | 1.2 mg | - |
| Propyl 4-hydroxy benzoate sodium | 0.2 mg | - |
| Methyl 4-hydroxy benzoate | - | 1.2 mg |
| Propyl 4-hydroxy benzoate | - | 0.2 mg |
| Citric acid | 0.65 mg | - |
| Banana flavor | 1 mg | 1 mg |
| Purified Water | Q.s to 1 mL | Q.s to 1 mL |

The aqueous solutions 5A and 5B of apixaban were obtained by a process similar to that disclosed in Example 2 above, with the only difference being that composition 5B did not contain citric acid, and an additional step of heating the solution to 80-85°C was included to ensure solubilization of Methyl 4-hydroxy benzoate and Propyl 4-hydroxy benzoate.

The compositions were stored at 60^{O}C for 15 days and tested for pH -

| | **Specification** | **Composition 5A** | | **Composition 5B** | |
|---|---|---|---|---|---|
| | | **Initial** | **60^{O}C/15 days** | **Initial** | **60^{O}C/15 days** |
| **pH** | 5.50-7.50 | 6.54 | 6.25 | 6.52 | 5.70 |

The study indicated that while the known, unknown and total impurities were within the specification, the pH of composition 5B, which does not contain citric acid, was found to lower. Since the pH for example 5A, which contains citric acid, remains fairly within acceptable levels of change during this period, it seems to indicate the relevance of citric acid in the aqueous apixaban compositions of the present invention.

### Example 6

Apixaban aqueous solutions with polyoxyl hydroxy stearate and sodium salts of methyl paraben and propyl paraben is provided in Table 6 below, with a target pH of 5.50 - 7.50.

**Table 6**

| **Ingredient** | **Quantity/mL** | |
|---|---|---|
| | **Composition 6A** | **Composition 6B** |
| Apixaban | 1 mg | 1 mg |
| Polyoxyl Hydroxy stearate (Kolliphor HS-15) | 40 mg | 40 mg |
| Ethanol | 100 µL | 100 µL |
| Propylene Glycol Vegetable | 340 µL | 340 µL |
| Glycerin | 60 µL | 60 µL |
| Sorbitol solution (Neosorb 70/02B) | 120 µl | 120 µl |
| Methyl 4-hydroxy benzoate sodium | 1.2 mg | - |
| Propyl 4-hydroxy benzoate sodium | 0.2 mg | - |
| Methyl 4-hydroxy benzoate | - | 1.2 mg |
| Propyl 4-hydroxy benzoate | - | 0.2 mg |
| Citric acid | 0.65 mg | - |
| Banana flavor | 1 mg | 1 mg |
| Bitter masker | 1 mg | 1 mg |
| Purified Water | Q.s to 1 mL | Q.s to 1 mL |

The aqueous solutions of apixaban composition were obtained by a process similar to that disclosed in Example 2 above, with the only difference being that composition 6B did not contain citric acid, and an additional step of heating the solution to 80-85°C was included to ensure solubilization of Methyl 4-hydroxy benzoate and Propyl 4-hydroxy benzoate.

The compositions were stored at 60^{O}C for 15 days and tested for the pH -

| | | **Specification** | **Composition 6A** | | **Composition 6B** | |
|---|---|---|---|---|---|---|
| | | | **Initial** | **60^{O}C/15 days** | **Initial** | **60^{O}C/15 days** |
| **pH** | | 5.50-7.50 | 6.38 | 6.12 | 7.04 | 6.61 |

The study indicated, again, that while the known, unknown and total impurities were within the specification, the pH of composition 6B, which does not contain citric acid, was found to fall significantly during the 15 days. The inventors expect the pH of example 6B to fall even further during extended storage. This experiment also emphasized the relevance of use of citric acid in the aqueous apixaban compositions of the present invention.

### Example 7 (Comparative study)

The aqueous solution of apixaban composition disclosed in US9452134B2 (see Table 7 below for ingredients - hereinafter referred to as "Reference Example 7") was reproduced as per the description provided therein.

**Table 7**

| **Ingredient** | **Quantity (mg/ml)** |
|---|---|
| Apixaban | 0.40 |
| Propylene Glycol | 164.81 |
| Glycerine | 235.45 |
| PEG 400 | 58.86 |
| Povidone K25 | 47.09 |
| Sodium lauryl sulfate | 5.89 |
| Fructose | 264.20 |
| Citric acid anhydrous | 235.45 |
| Sodium citrate dihydrate | 0.150 |
| Sucralose | 0.067 |
| Orange flavor | 4.72 |
| Methyl paraben | 14.75 |
| Propyl paraben | 0.909 |
| Purified water | 0.100 |

Nasogastric tube (NG tube) study was conducted to analyse the rate of reduction in apixaban present in the oral solution of Reference Example 7 and Example 2 of the present invention, when passed through the tube. Nasogastric tubes are types of medical catheters that deliver substances to the stomach via oesophagus by inserting the tube into the nose. In the present study, Polyvinyl chloride NG tubes which are open ended with a size (external) diameter of 12 French and a length of 125 cm were used.

Table 7A below provides results of the study:

**Table 7A**

| | **Type of NG tube** | **Reference Example 7** | | **Example 2** | |
|---|---|---|---|---|---|
| | | **Assay** | **Reduction in assay** | **Assay** | **Reduction in Assay** |
| **Initial assay** | - | 100.0 | NA | 100.5 | NA |
| **Assay-1** | PVC make, 12 French, 125 cm, open ended. | 93.9 | 5.7 | 99.1 | 1.4 |
| **Assay-2** | | 93.6 | 6 | 98.9 | 1.6 |
| **Assay-3** | | 90.9 | 8.5 | 98.7 | 1.8 |

Thus, it can be seen that the apixaban oral solution of the present invention is superior to Reference Example 7 in terms of maintaining the assay when administered through NG tube, thereby providing the additional ability to administer the solution in a safe and efficacious manner to patients that may be bed ridden, or that need to be administered the solution through NG tube for any reason.

### Example 8

NG tube study was also conducted for crushed tablets of Bristol Myers Squibb Co. (BMS) (commercially available under the brand name ELIQUIS^{®}). In the present study, Polyvinyl chloride NG tubes which are open ended with a size (external) diameter of 12 French and a length of 125 cm was used. The results of the assay are tabulated in Table 8.

**Table 8**

| | **Type of NG tube** | **ELIQUIS^{®} (GX7608)** | **Reduction in Assay** |
|---|---|---|---|
| **Initial assay** | NA | 98.0 | NA |
| **Sample-1** | PVC make, 12 French, 125 cm, open ended | 97.3 | 0.7 |
| **Sample -2** | | 95.6 | 2.4 |
| **Sample -3** | | 97.2 | 0.8 |
| **Sample -4** | | 98.0 | 0 |
| **Sample -5** | | 96.8 | 1.2 |
| **Sample -6** | | 97.5 | 0.5 |

As can be seen in Table 8 above, using crushed Eliquis^{®} tablet and administering the same through NG tube, as described on the Eliquis^{®} label, results in fall in assay of apixaban. Such observation is not seen for the apixaban oral solution of the present invention, as described in Example 7, Table 7A above. Thus, the apixaban oral solution of the present invention is superior to prior known use of crushed apixaban tablets.

### Example 9

The aqueous solution of apixaban as provided in Table 9 below was obtained by a process similar to that disclosed in Example 2 above.

**Table 9**

| **Ingredients** | **Quantity** | **% w/w** |
|---|---|---|
| Apixaban | 1 g | 0.10 |
| Polyoxyl 35 castor oil | 80 g | 8.00 |
| Ethanol | 100 mL | 10.00 |
| Propylene Glycol | 300 mL | 30.00 |
| Glycerin | 60 mL | 6.00 |
| Sorbitol solution | 120 mL | 12.00 |
| Sodium benzoate | 1 g | 0.10 |
| Malic acid | 6.75 g | 0.675 |
| Banana flavor | 1 g | 0.10 |
| Purified Water | Q.s to 1 L | Q.s to 100.00 |

### Example 10

The aqueous solution of apixaban as provided in Table 10 below was obtained by a process similar to that disclosed in Example 2 above.

**Table 10**

| **Ingredients** | **Quantity** | **% w/w** |
|---|---|---|
| Apixaban | 1.5 g | 0.10 |
| Polyoxyl 15 Hydroxystearate | 60 g | 4.00 |
| Ethanol | 150 mL | 10.00 |
| Propylene Glycol | 510 mL | 34.00 |
| Glycerin | 90 mL | 6.00 |
| Sorbitol solution | 180 mL | 12.00 |
| Sodium benzoate | 1.5 g | 0.10 |
| Malic acid | 10.125 g | 0.675 |
| Banana flavor | 1.5 g | 0.10 |
| Bitter masker | 1.5 g | 0.10 |
| Purified Water | Q.s to 1.5 L | Q.s to 100.00 |

### Example 11

The aqueous solution of apixaban as provided in Table 11 below was obtained by a process similar to that disclosed in Example 2 above.

**Table 11**

| **Ingredients** | **Quantity** | **Quantity (% w/w)** |
|---|---|---|
| Apixaban | 1.5 g | 0.10 |
| Polyoxyl 15 Hydroxystearate | 60 g | 4.00 |
| Ethanol | 150 mL | 10.00 |
| Propylene Glycol | 510 mL | 34.00 |
| Glycerin | 90 mL | 6.00 |
| Sorbitol solution | 180 mL | 12.00 |
| Sodium benzoate | 1.5 g | 0.10 |
| Tartaric acid | 6.21 g | 0.41 |
| Banana flavor | 1.5 g | 0.10 |
| Bitter masker | 1.5 g | 0.10 |
| Purified Water | Q.s to 1.5 L | Q.s to 100.00 |

### Example 12

Table 12 below provides the assay, pH and impurity profile of apixaban oral liquid compositions of Examples 9, 10 and 11.

**Table 12**

| **Parameter** | **Example 9** | **Example 10** | **Example 11** |
|---|---|---|---|
| **pH** | 3.51 | 3.62 | 3.55 |
| **Assay** | 100.0 | 99.2 | 100.7 |
| **Preservative content** | 100.73 | 100.16 | 98.28 |

| **Related substances** | | | |
|---|---|---|---|
| **Impurity A** | ND | ND | ND |
| **Impurity B** | ND | ND | ND |
| **Impurity C** | 0.02 | 0.01 | 0.02 |
| **Impurity D** | ND | ND | ND |
| **Unknown impurities** | 0.01 | ND | ND |
| **Total Impurities** | 0.02 | 0.02 | 0.02 |

| | | | |
|---|---|---|---|
| ND : Not Detected | | | |

The above table indicates that the apixaban oral solutions containing malic acid and tartaric acid are stable and contain low levels of impurities.

## Claims

1. An aqueous apixaban solution formulation, comprising
a. 1 mg/ml of apixaban,
b. a vehicle selected from one or more of water, polyoxyl 35 castor oil, macrogol-15-hydroxystearate, ethanol, propylene glycol, glycerine and sorbitol, preferably a vehicle comprising water in admixture with one or more of polyoxyl 35 castor oil, macrogol-15-hydroxystearate, ethanol, propylene glycol, glycerine and sorbitol,
c. optionally, a preservative selected from methyl paraben, propyl paraben, methyl paraben sodium, propyl paraben sodium, methyl 4-hydroxy benzoate, propyl 4-hydroxy benzoate and mixtures thereof,
d. an organic acid in an amount of about 0.01 mg/ml to about 10 mg/ml,
wherein, preferably, upon storage at 40⁰C/75% RH for up to 6 months, the amount of apixaban in the composition is NLT 98%.

2. A stable aqueous solution composition comprising:
a. 1 mg/ml of apixaban,
b. vehicle selected from one or more of water, polyoxyl 35 castor oil, macrogol-15-hydroxystearate, ethanol, propylene glycol, glycerine and sorbitol,
c. optionally, a preservative selected from methyl paraben, propyl paraben, methyl paraben sodium, propyl paraben sodium, methyl 4-hydroxy benzoate, propyl 4-hydroxy benzoate and mixtures thereof,
d. an organic acid in an amount of about 0.01 mg/ml to about 10 mg/ml,
wherein upon storage at 40⁰C/75% RH for up to 6 months, the amount of apixaban in the composition is NLT 99.5%.

3. The composition as claimed in claim 1 or 2, wherein the organic acid is selected from the group comprising citric acid, malic acid, tartaric acid, phosphoric acid, glutamic acid, lactic acid, succinic acid, pimelic acid, adipic acid, ascorbic acid, fumaric acid, acetic acid and mixtures thereof.

4. The composition as claimed in claim 1 or 2, further comprising 0.1% to about 10% (w/w) of a preservative.

5. The composition as claimed in claim 4, wherein composition contains one or more preservatives selected from the group comprising methyl paraben, methyl paraben sodium, ethyl paraben, propyl paraben, propyl paraben sodium, benzoic acid, sorbic acid, sodium benzoate, methyl 4-hydroxy benzoate, propyl 4-hydroxy benzoate and mixtures thereof.

6. The composition as claimed in claim 1 or 2, further comprising one or more agents selected from a flavoring agent, an antioxidant, a sweetener and a buffer.

7. The composition as claimed in claim 6, wherein the flavoring agent is selected from a group comprising orange flavor, cherry flavor, strawberry flavor, bubblegum flavor, grape flavor, lemon flavor, banana flavor, mango flavor, pineapple flavor, mint flavor, mixed fruit flavor, vanilla flavor, tutti-fruity flavor, fruit punch flavor, and mixtures thereof.

8. The composition as claimed in claim 6, wherein the antioxidant is selected from a group comprising ascorbic acid, butylated hydroxy anisole, butylated hydroxytoluene, vitamin E, vitamin E PEG 1000 succinate, and mixtures thereof.

9. The composition as claimed in claim 6, wherein the sweetener is selected from a group comprising sucralose, aspartame, acesulfame potassium, saccharin sodium, sucrose, fructose, high fructose corn syrup, lactose, dextrose, and mixtures thereof.

10. The composition as claimed in claim 6, wherein the buffer is selected from a group comprising malic acid, tartaric acid, phosphoric acid, glutamic acid, lactic acid, succinic acid, pimelic acid, adipic acid, ascorbic acid, tosylic acid, benzenesulfonic acid, suberic acid, alginic acid, fumaric acid, acetic acid, citrate buffer, acetate buffer, phosphate buffer, and mixtures thereof.

11. The composition a claimed in claim 1 or 2, wherein the composition has a pH in the range of from about 3.5 to about 8.

12. The composition as claimed in claim 1 or 2, wherein the composition is stable for a period of 24 months.

13. The composition as claimed in claim 1 or 2, for use in manufacture of a medicament in treating or preventing a disease or a condition.

14. The composition as claimed in claim 13, wherein the condition or the disease is selected from a group consisting of thromboembolic disorder, stroke, and systemic embolism in nonvalvular atrial fibrillation, prophylaxis of deep vein thrombosis, deep vein thrombosis, and pulmonary embolism.

15. The composition as claimed in claims 13 and 14, wherein the composition is administered orally, through nasogastric tube or through gastronomy tube using a dosing syringe.
